# EUROPEAN PATENT APPLICATION

(11) **EP 2 796 169 A1**
(43) Date of publication of application: **29.10.2014**
(21) Application number: 14166114.0
(22) Date of filing: 25.04.2014
(51) Int. Cl.: A61N 5/10

(54) **Bendable, shielding brachytherapy needle holder providing one-handed access**

(30) Priority: 28.04.2013 US 201361816770 P
(71) Applicant: Bard Brachytherapy Inc., Carol Stream, IL 60188 (US)
(72) Inventor: Drobnik, Christopher, Wauconda, IL Illinois 60084 (US); Spittle, John, Volo, IL Illinois 60020 (US); Krachon, Mike, Atlanta, GA Georgia 30318 (US); Riescher, Russ, Loganville, GA Georgia 30052 (US)
(74) Representative: Curley, Donnacha John

(57) **Abstract**

A brachytherapy needle holder includes a needle card holding one or more brachytherapy needles preloaded with one or more brachytherapy seeds, wherein the needle holder includes a scored needle card shield that may be bent at the score so that the needle card may stand up on its own, providing one-handed access. A brachytherapy needle holder includes a needle card holding one or more brachytherapy needles preloaded with one or more brachytherapy seeds, wherein the needle holder further includes a needle card shield that provides one or more of rigidity and shielding from radiation for one or more of a physician and other medical personnel.

## Description

### PRIORITY CLAIM

The present application claims the priority benefit of U.S. provisional patent application number 61/816,770 filed April 28, 2013 and entitled "Bendable, Shielding Brachytherapy Needle Tray Providing Easy Needle Access," the disclosure of which is incorporated herein by reference.

### SUMMARY

A brachytherapy needle holder includes a needle card holding one or more brachytherapy needles preloaded with one or more brachytherapy seeds, wherein the needle holder includes a scored needle card shield that may be bent at the score so that the needle holder can stand up on its own, providing one-handed access.

A brachytherapy needle holder includes a needle card holding one or more brachytherapy needles preloaded with one or more brachytherapy seeds, wherein the needle holder further includes a needle card shield that provides one or more of rigidity and shielding from radiation for one or more of a physician and other medical personnel.

The application is to be taken to extend to the followed numbered statements:
1. A brachytherapy needle holder comprising a needle card holding one or more brachytherapy needles preloaded with one or more brachytherapy seeds, wherein the needle holder comprises a scored needle card shield that may be bent at the score so that the needle card may stand up on its own, providing one-handed access.
2. The brachytherapy needle holder of statement 1, wherein the needle card comprises one or more corrugations configured to hold the one or more brachytherapy needles securely in place.
3. The brachytherapy needle holder of statement 2, wherein the one or more brachytherapy needles are held within one or more of flutes and channels in the needle holder.
4. The brachytherapy needle holder of statement 3, wherein the one or more brachytherapy needles are held loosely within the one or more of flutes and channels to facilitate ease of removal.
5. The brachytherapy needle holder of statement 1, wherein the needle card shield comprises a stenciled manufacturer name.
6. The brachytherapy needle holder of statement 5, wherein the stenciled manufacturer name removes material from the needle holder, thereby reducing the weight of the needle holder.
7. The brachytherapy needle holder of statement 2, wherein the needle card is die-cut.
8. The brachytherapy needle holder of statement 2, wherein the needle card comprises one or more of polyethylene, polypropylene, and polycarbonate.
9. The brachytherapy needle holder of statement 1, wherein the needle card is configured to facilitate removal of one or more brachytherapy needles using one hand during the implanting of the seeds in a patient.
10. The brachytherapy needle holder of statement 1, wherein the needle card shield provides one or more of rigidity and shielding from radiation.
11. The brachytherapy needle holder of statement 1, wherein the needle card shield further comprises tape configured to retain the brachytherapy needles in place.
12. The brachytherapy needle holder of statement 10, wherein the tape comprises foam tape.
13. A method for treating a brachytherapy patient, comprising the steps of:
   receiving a needle holder comprising a needle card holding one or more brachytherapy needles preloaded with one or more brachytherapy seeds, wherein the needle holder comprises a scored needle card shield that may be bent at the score;
   bending the needle card shield along the score;
   standing the needle card up;
   removing one or more of the brachytherapy needles from the needle card; and
   implanting in the patient at least one of the one or more brachytherapy seeds from the one or more brachytherapy needles.
14. The method of statement 13, wherein the step of standing up comprises standing the needle card up so that it stands up separately from the needle card shield, providing one-handed access.
15. The method of statement 13, wherein the step of removing comprises removing at least one of the one or more of the brachytherapy needles with one hand.
16. The method of statement 13, wherein the step of implanting comprises implanting at least one of the one or more of the brachytherapy seeds with one hand.
17. The method of statement 13, comprising the step of shielding oneself from radiation using the needle card shield.
18. A brachytherapy needle holder comprising a needle card holding one or more brachytherapy needles preloaded with one or more brachytherapy seeds,
wherein the needle holder further comprises a needle card shield that provides one or more of rigidity and shielding from radiation for one or more of a physician and other medical personnel.

### DESCRIPTION OF THE DRAWINGS

Figure 1A is a drawing showing the components of the apparatus and the assembly of the apparatus.
Figure 1B is a drawing showing a more detailed view of a first shipping insert as comprised in the apparatus and components thereof.
Figure 1C is a drawing showing an exploded view of a needle box carton comprising a needle box, in turn comprising a needle holder assembly and components thereof.
Figure 2A is a drawing showing a needle holder assembly comprising a loaded needle holder, a top needle tray, a bottom needle tray, and a needle tray lid.
Figure 2B is a drawing showing an exploded view of a needle holder and components thereof.
Figure 2C is a drawing showing a needle holder comprising a needle card shield and a needle card, the needle card shield comprising a score at a location where the needle card shield may be bent in order to use it as a stand for the needle holder.
Figure 2D is a drawing showing a needle holder after the needle card shield has been bent along the score and used as a stand for the needle holder.
Figure 3 is a flowchart of a method for treating a brachytherapy patient.

### DETAILED DESCRIPTION

Cancer is one of the leading health problems and causes of mortality and morbidity in the United States and worldwide. Cancers are commonly treated using radiation therapy, which employs radiation to kill cancer cells. Prostate cancer is a serious form of cancer that affects many men, particularly as their age increases to middle age and beyond.

Brachytherapy treatment is a localized method of treating cancer in which a radiation source is placed inside or next to the area requiring treatment. An advantage of brachytherapy is that very high doses of ionizing radiation may be delivered to a localized area, such that the radiation may be supplied primarily to the area to be treated, without significantly harming tissues elsewhere in the body. This advantage, combined with a rapid reduction in radiation dose as a function of distance, may shield distant parts of the anatomy from one or more of unwanted radiation and unneeded radiation. Brachytherapy has provided excellent results for localized control of various tumors.

Brachytherapy treatment is most commonly employed to treat one or more of prostate cancer, cervical cancer, breast cancer, and skin cancer. One common example of a brachytherapy radiation source is radioactive seeds. The radioactive seeds may be deployed in needles. The radioactive seeds may be implanted in the prostate gland to kill prostate cancer cells.

To evaluate a patient's medical condition, a physician may employ tools such as one or more of ultrasound, computerized axial tomography (CAT) scans, and X-ray images in concert with dose-planning computer software programs. The physician may construct an optimal treatment plan to distribute radiation as evenly as possible throughout the affected tissue.

Proper seed placement may be important for controlling the distribution of radiation throughout the prostate gland. Placing the radioactive seeds too close together may result in a portion of the prostate receiving more radiation than is optimal. Not placing enough seeds in portions of the tissue may result in a portion of the prostate receiving less radiation than is optimal. For example, not placing enough seeds in a portion of the tissue may result in that portion of the prostate receiving an amount of radiation that may be insufficient to kill the cancer cells in that portion.

The physician may insert seeds of the same or different radioactive strengths in pre-selected locations of the tissue. The seeds may be inserted into the tissue through an implantation needle, also known as a brachytherapy needle. Multiple brachytherapy needles may be required to insert the seeds into multiple locations of the tissue. One or more of the brachytherapy needles may insert one or more seeds. An average brachytherapy session may involve the implantation of approximately eighty to one hundred seeds.

Modern medicine carries many pressures, including economic pressures, pressures relating to time demands, and otherwise. Simply put, doctors' time is very valuable, as is the time of other medical personnel, and any available product or method for reducing these time demands and increasing the efficiency with which doctors and other medical personnel can treat patients is potentially lucrative. Accordingly, products and methods that improve the efficiency with which physician and other medical personnel can treat patients may be highly valuable.

Products and methods that promote the safety of physicians and other medical personnel are also quite valuable. Given their value, physicians and other medical personnel who may be compelled to miss work due to illness may cost their employer substantial sums of money. The potential value of an invention that can promote the safety of physicians and other medical personnel may be even greater when the protection of the physicians and other medical personnel is integrated in an invention that also promotes increases efficiency of treatment.

In addition to the preloaded needles, according to embodiments of the invention, the shipping box may also contain one or more of the following:

1) Extra needles. The extra needles may, according to other embodiments of the invention, be held in open positions on the plastic insert needle cards.

2) A calibrated seed. For example, according to still further embodiments of the invention, a calibrated seed may be positioned in a screw-cap vial within a steel shield, and the steel shield may be comprised in a film-film pouch. Additional linking components or spacing elements can be provided in foil-film or film-film pouches.

3) One or more loose seeds. For example, according to yet further embodiments of the invention, the one or more loose seeds may be positioned within a screw-cap vial. According to yet other embodiments of the invention, the screw-cap vial may be comprised in a container. According to yet other embodiments of the invention, the container may provide one or more of radiation shielding and protection during shipment. According to further embodiments of the invention, the container may be a steel container. According to other embodiments of the invention, the container may be a stainless steel container. According to still more embodiments of the invention, the container may be a screw-cap container. According to still further embodiments of the invention, the container may be a screw-cap stainless steel container. According to embodiments of the invention, the stainless steel container may be comprised in a film-film pouch. According to yet more embodiments of the invention, the film-film pouch may act as a sterile barrier.

4) A pouch comprising one or more of synthetic spacers and linking components. For example, a pouch comprising one or more of SourceLink™ components and SourceLink™ spacers manufactured by C.R. Bard, Inc. of Murray Hill, New Jersey.

5) One or more loaded cartridges. For example, according to yet other embodiments of the invention, the pouch may comprise one or more Mick cartridges, manufactured by Mick Radio-Nuclear Instruments of Mount Vernon, New York (www . micknuclear . com). According to further embodiments of the invention, one or more of the one or more cartridges may be held within a steel shield, which may in turn be comprised in a film-film pouch. According to still further embodiments of the invention, one or more of the one or more loaded cartridges may be used to deploy seeds into a human patient for brachytherapy treatment. According to yet other embodiments of the invention, one or more of the one or more loaded cartridges may be used to deploy one seed at a time into a human patient for brachytherapy treatment.

According to embodiments of the invention, the shipping box may be designed to hold a single steel needle card containing pre-loaded needles. For example, the shipping box may be designed to hold a single needle box which in turn holds up to four sealed needle card assemblies, each of which may comprise up to ten needles. According to yet further embodiments of the invention, the shipping box may be configured to hold two loose seed shields in pouches, six loaded cartridges in individual shields/pouches, and up to ten spacer pouches.

According to embodiments of the invention, the steel needle container may be shipped to the customer in a carton that may comprise easy pull tabs. According to yet other embodiments of the invention, the easy pull tabs may facilitate the customer's removal of the steel needle container from the shipping carton. According to still further embodiments of the invention, a customer may open the package and use the pull tabs to remove the needle container from the shipping carton. According to yet other embodiments of the invention, the steel needle container may provide one or more of stability, product protection and shielding of radiation during shipment.

According to other embodiments of the invention, the steel container may be easily opened. According to still other embodiments of the invention, the steel container may comprise between one and four needle cards. According to yet other embodiments of the invention, one or more of the needle card assemblies may be safety sealed. According to still further embodiments of the invention, the customer may slide the container out of the carton.

According to further embodiments of the invention, the needles in one or more needle cards may be visually verified within the sterile needle card assembly prior to a customer breaking the sterile barrier by opening a seal of the needle card assembly.

According to other embodiments of the invention, the customer may break the seal on the needle card assembly. According to still further embodiments of the invention, the customer may peel back a needle card lid of the needle card assembly. According to yet other embodiments of the invention, the customer may ensure that the lid is fully pulled back in order to ensure customer access to the needle holder.

According to yet other embodiments of the invention, a retention insert may cover the needles in order to do one or more of further protect them, further hold them in place, further enhance the integrity of the sterile barrier and further ensure their stability. According to embodiments of the invention, the retention insert may be comprised of transparent plastic. According to further embodiments of the invention, the customer may remove the retention insert in order to expose the needle card assembly. According to embodiments of the invention, the customer may place the needle card assembly in the sterile field.

According to embodiments of the invention, the needle card assembly may be one of partly recyclable and fully recyclable. According to yet other embodiments of the invention, the needle card assembly may be one of partly recyclable and fully recyclable at the point of use. According to still further embodiments of the invention, the needle card assembly may be one of partly and fully lead-free. According to yet further embodiments of the invention, the needle card assembly may be substantially lead-free.

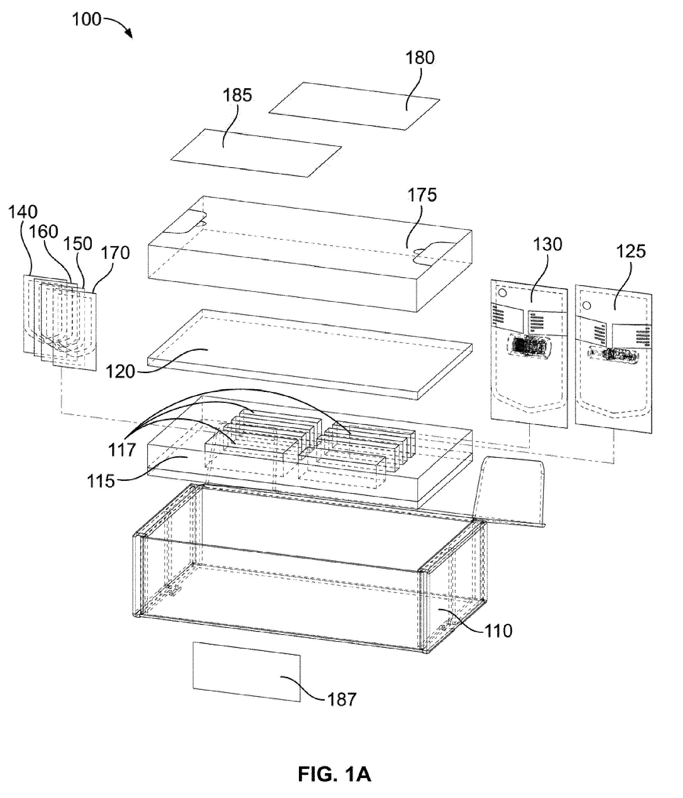

Figure 1A is a drawing showing the components of the apparatus 100 and the assembly of the apparatus 100. The components of the apparatus 100 include a shipping box 110, a first shipping insert 115 comprising one or more cavities 117, a second shipping insert 120, one or more loose seed cartridges 125, one or more loose seed vials 130, one or more first fixed seed spacers 140, one or more second fixed seed spacers 150, one or more extension seed spacers 160, one or more non-connecting seed spacers 170, a needle box carton 175, a document packet 180 containing information that is provided to the customer to describe one or more of product usage and product attributes (e.g. certificates of analysis), a return packet 185 to enable customers to return unused seeds to the original manufacturer if desired and packaging tape 187.

The shipping box 110 has the approximate shape of a right parallelepiped or "brick." The shipping box 110 is configured to comfortably, snugly hold the components of the apparatus 100 while minimizing one or more of overall dimensions and weight. One or more of shipping costs and material usage may thereby be reduced. For example, one possible set of dimensions of the shipping box 110 depicted in Figure 1A is approximately 19.50 inches or approximately 49.53 centimeters (cm) in length, approximately 10.21 inches or approximately 25.93 cm in width, and approximately 5.38 inches or approximately 1.67 cm in depth.

The first shipping insert 115 can have the approximate shape of a right parallelepiped or "brick." The first shipping insert 115 is configured to be inserted snugly into shipping box 110. The first shipping insert 115 may comprise foam or a similar material.

The first shipping insert 115 comprises one or more cavities. Figure 1A depicts an example in which the first shipping insert 115 comprises eight cavities 117.

In other embodiments, the first shipping insert 115 may comprise a different number of cavities.

Similarly, the second shipping insert 120 is configured to be inserted snugly into shipping box 110. Commonly, but not necessarily, first shipping insert 115 and second shipping insert 120 will have approximately equal widths. Commonly, but not necessarily, first shipping insert 115 and second shipping insert 120 will have approximately equal lengths.

Accordingly, first shipping insert 115 has dimensions that are less than the dimensions of shipping box 110. Similarly, second shipping insert 120 has dimensions that are less than the dimensions of shipping box 110. As depicted, for example, first shipping insert 115 has approximate dimensions of 19 inches in length, 9.5 inches in width, and 2 inches in depth. As depicted, for example, second shipping insert 120 has approximate dimensions of 19 inches in length, 9.5 inches in width, and .5 inches in depth.

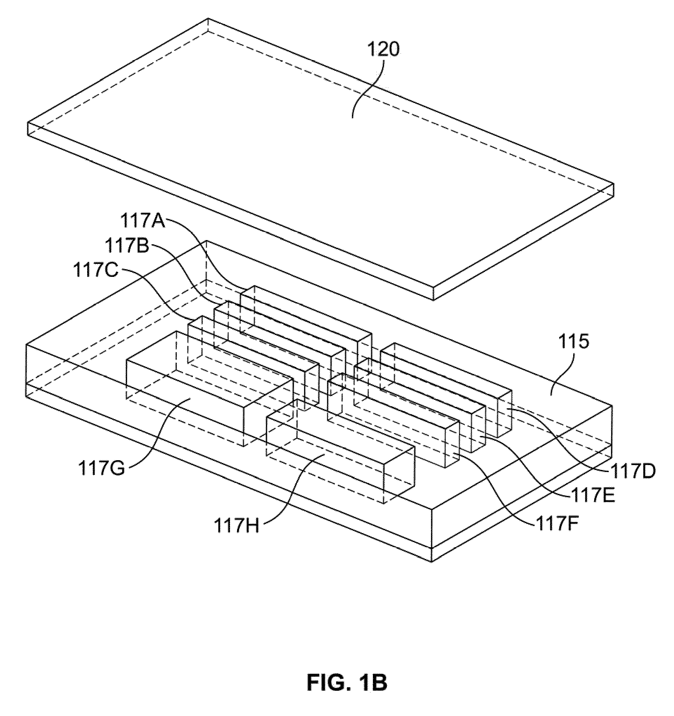

Figure 1B is a drawing showing a more detailed view of a first shipping insert as comprised in the apparatus and components thereof.

Referring to Figure 1B, as depicted, for example, the first shipping insert 115 comprises eight cavities 117A-117H ,all of which, for example, have approximate dimensions of 19 inches in length, and all of which are approximately 1.5 inches in depth. As depicted, for example, six of the cavities 117A-117F have widths of approximately 0.75 inches, cavity 117G has a width of approximately 1.5 inches, and cavity 117H has a width of approximately 1.5 inches. Also depicted in Figure 1B is the second shipping insert 120 that is placed on top of the first shipping insert 115 when the shipping box 110 is prepared.

While the cavities 117A-117H may have all the same dimensions, when the cavities 117A-117H do not have all the same dimensions, it may be so that the various differently sized cavities 117A-117H may accommodate one or more of other containers of seeds and related components used in the brachytherapy procedure characterized by one or more of different types and different sizes.

For example, one or more of cavities 117A-117F may be configured to snugly hold in place one or more of a shielded magazine comprising one or more radioactive brachytherapy seeds and a shielded cartridge comprising one or more radioactive brachytherapy seeds. For example, the one or more of a cartridge and a magazine may be configured to fit into a gun apparatus configured to dispense the brachytherapy seeds into an appropriate location for brachytherapy treatment of a patient. For example, the one or more of a cartridge and a magazine may comprise approximately fifteen seeds.

For example, one or more of cavities 117G and 117H may be configured to snugly hold in place a shielded vial comprising one or more loose brachytherapy seeds. For example, the shielded vial may comprise up to approximately 900 brachytherapy seeds. For example, since in this example, cavity 117G has a larger width than does cavity 117H, cavity 117G may snugly hold in place a larger vial that can hold more seeds.

The first fixed seed spacers 140 comprise one or more pre-loaded brachytherapy needles (not shown). One or more of the one or more first fixed seed spacers 140 may be bio-absorbable. According to embodiments of the invention, one or more of the one or more brachytherapy seeds pre-loaded into the brachytherapy needles may be assembled into a seed train with desired spacing using one or more of one or more first fixed seed spacers 140, one or more second seed spacers 150, one or more extension seed spacers 160, and one or more non-connecting seed spacers 170. The spacing between adjacent radioactive brachytherapy seeds may be, for example, approximately 1.0 centimeter.

One or more of the pre-loaded brachytherapy needles (not shown) comprises one or more radioactive brachytherapy seeds connected together in a desired order. Connecting the seeds together in a desired order may help reduce the migration of seeds in the body, thereby helping to maintain control of the treatment. Typically, though not necessarily, one or more of the one or more first fixed seed spacers 140, one or more second seed spacers 150, and one or more extension seed spacers 160 may comprise a plurality of radioactive seeds that are separated.

The second fixed seed spacers 150 comprise one or more pre-loaded brachytherapy needles (not shown). One or more of the one or more second fixed seed spacers 150 may be bio-absorbable. According to embodiments of the invention, one or more of the one or more seeds comprised in the pre-loaded brachytherapy needles may be assembled into a seed train with desired spacing using one or more of the second fixed seed spacers 150. The spacing between adjacent radioactive brachytherapy seeds for one or more of the second fixed seed spacers 140 may be, for example, approximately 0.5 centimeters. One or more of the pre-loaded brachytherapy needles comprises one or more radioactive brachytherapy seeds connected together in a desired order. Connecting the seeds together in a desired order may help reduce the migration of seeds in the body, thereby helping to maintain control of the treatment. Typically, though not necessarily, one or more of the second fixed seed spacers 150 may comprise a plurality of radioactive seeds that are separated.

The extension seed spacers 160 comprise one or more pre-loaded brachytherapy needles (not shown). One or more of the one or more extension seed spacers 160 may be bio-absorbable. According to embodiments of the invention, one or more of the one or more seeds comprised in the pre-loaded brachytherapy needles may be assembled into a seed train with desired spacing using one or more of the extension seed spacers 160. The spacing between adjacent radioactive brachytherapy seeds for one or more of the extension seed spacers 160 may be adjusted according to the user's needs.

One or more of the pre-loaded brachytherapy needles comprises one or more radioactive brachytherapy seeds connected together in a desired order and with desired spacing. Connecting the seeds together in a desired order may help reduce the migration of seeds in the body, thereby helping to maintain control of the treatment. Typically, though not necessarily, one or more of the extension seed spacers 160 may comprise a plurality of radioactive seeds that are separated.

The non-connecting seed spacers 170 comprise one or more pre-loaded needles (not shown). One or more of the one or more non-connecting seed spacers 170 may be bio-absorbable. One or more of the one or more non-connecting seed spacers 170 may be convenient for application of one or more radioactive brachytherapy seeds adjacent to a patient's urethra. One or more of the one or more non-connecting seed spacers 170 may be convenient for application of one or more radioactive brachytherapy seeds in applications other than adjacent to a patient's urethra. For example, one or more of the non-connecting seed spacers 170 comprise one or more pre-loaded brachytherapy needles. According to embodiments of the invention, one or more of the one or more pre-loaded brachytherapy needles may be assembled into a seed train with spacing using one or more of the non-connecting seed spacers 170. The spacing between adjacent radioactive brachytherapy seeds for one or more of the non-connecting seed spacers 170 may be adjusted according to the user's needs.

One or more of the pre-loaded brachytherapy needles comprises one or more radioactive brachytherapy seeds connected together in a desired order and with desired spacing. Connecting the seeds together in a desired order may help reduce the migration of seeds in the body, thereby helping to maintain control of the treatment. Typically, though not necessarily, one or more of the non-connecting seed spacers 170 may comprise a plurality of radioactive seeds that are separated. Typically, though not necessarily, one or more of the non-connecting seed spacers 170 may comprise a line that comprises a seed, a spacer, a seed a spacer. Typically, though not necessarily, one or more of the non-connecting seed spacers 170 may comprise a monofilament line. For example, two adjacent brachytherapy seeds comprised in one of the one or more non-connecting seed spacers 170 may be separated by approximately 5.5 millimeters.

Accordingly, the needle box carton 175 has dimensions that are less than the dimensions of shipping box 110. As depicted, for example, the needle box carton 175 has approximate dimensions of 19 inches in length, 9.5 inches in width, and 2 inches in depth.

The document packet 180 may comprise one or more of a user's manual, specifications, certificates of analysis, medical provider advice, medical billing information, contact information for the manufacturer, a product brochure, a customer satisfaction questionnaire, and other documents relating to the product.

The return packet 185 may comprise one or more of a return shipping label, a return authorization form, instructions for making a return, and other information relating to a return.

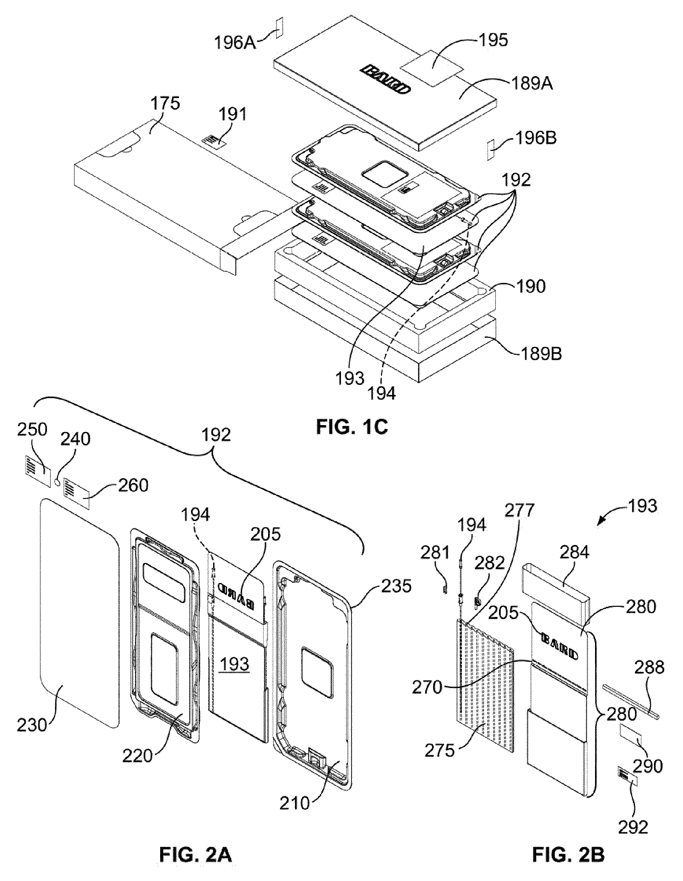

Figure 1C is a drawing showing an exploded view of the needle box carton 175 comprising the needle box, in turn comprising the needle holder assembly 192 and components thereof.

Referring to Figure 1C, the needle box carton 175 may comprise one or more needle boxes 189. As depicted, the needle box 189 comprises a needle box top 189A and a needle box bottom 189B. A needle box insert 190 fits into the needle box bottom 189B. The needle box carton 175 may optionally comprise needle box carton labels 191. At least one of the one or more needle boxes 189 comprises one or more needle card assemblies 192 comprising one or more needle holders 193. The needle box insert is configured to hold the one or more needle card assemblies 192. In Figure 1C, a single needle card assembly 192 is depicted. One or more of the needle holders 193 comprises one or more brachytherapy needles 194. The depicted example shows a single needle holder 193. The needle box top 189A may comprise a needle box label 195. The needle box 189 may optionally comprise one or more of a first needle box tape 196A and a second needle box tape 196B.

Figure 2A is a drawing showing an exploded view of the needle holder assembly 192 comprising the needle holder 193, a bottom needle tray 210, a top needle tray 220, and a needle tray lid 230.

The needle holder assembly 192 comprises one or more needle holders 193. The depicted example shows a single needle holder 193 comprising one or more brachytherapy needles 194. The needle holder 193 may comprise a manufacturer name 205. The manufacturer name 205 may be printed on the needle holder 193. The manufacturer name 205 may be embossed on the needle holder 193. The manufacturer name 205 may be stenciled on the needle holder 193. As depicted in Figure 2A, the manufacturer name 205 is stenciled on the needle holder 193.

The needle holder 193 may be protected and held snugly in place by a bottom needle tray 210. The bottom needle tray 210 may comprise a flange 235 extending around the outer edge of the bottom needle tray 210. The needle tray lid 230 may be heat sealed to the flange 235 to promote sterility. The needle holder 193 may be wrapped in plastic to promote sterility and consumer confidence in the integrity of the needles 194. The needle holder 193 may be protected and held snugly in place by a top needle tray 220.

One or more of the needle holder 193 and the top needle tray 220 may rest on a needle tray lid 230. The needle tray lid 230 may comprise one or more of a gamma ray indicator 240, a first needle card label 250, and a second needle card label 260. The gamma ray indicator 240 may indicate gamma ray exposure to one or more of physicians and other medical personnel in order to indicate the product has been gamma sterilized. The needle holder 193 may comprise a score 270.

One or more of the first needle card label 250 and the second needle card label 260 may comprise one or more of dosage information, an order number, a product number, manufacturer contact information, date of manufacture, location of manufacture, relevant intellectual property information including relevant information on one or more of pending patent applications and issued patents, and information on one or more of the sterility and sterilant exposure of the product.

Figure 2B is a drawing showing an exploded view of a needle holder 193 and components thereof. The needle holder 193 comprises a needle card 275 comprising one or more brachytherapy needles 194. Commonly, and as depicted in Figure 2B, though not necessarily, the needle card 275 will be a corrugated needle card 275. Commonly, and as depicted in Figure 2B, though not necessarily, the needle holder comprises one or more of flutes 277 and channels 277. Commonly, though not necessarily, the needles 194 may be held loosely in the one or more of flutes 277 and channels 277 to facilitate ease of removal. The needle holder 193 further comprises a needle card shield 280.

The one or more brachytherapy needles 194 are loaded into the needle card 275. The needle card 275 may have corrugations configured so as to hold the brachytherapy needles 194 securely in place yet render them easily removable by one or more of a physician and other medical practitioners using a single hand. The needle card shield 280 and the needle card 275 are fastened together using one or more of an adhesive and another attachment method, thereby forming the needle holder 193.

The needle holder 193 may be configured to stand up on its own. The needle card shield 280 may comprise the score 270. The score 270 may be positioned parallel to the short edge of the needle card shield 280 so that it runs across the short side of the needle card shield 280. The score 270 may be positioned at a location where the needle card shield 280 may be bent in order to use it as a stand. For example, the score 270 may be located about one-third of the way from the top of the needle card shield 280 so that the needle holder 193 may stand up on its own. According to still further embodiments of the invention, the needle stand may be configured to facilitate easy one-handed access to one or more brachytherapy needles 194 comprised in the needle holder 193.

When the needle card 275 is bent, the needle card 275 may be bent away from the needle card shield 280 so that the needles 194 may be more easily removed.

The needle 194 may further comprise a hub label 281. One or more of the needles 194 may be locked into place with a stylet lock 282 positioned at the top of the one or more needled 194. The needle holder 193 may further comprise a shield shrink wrap 284 that is positioned laterally around the needle card shield 280 to do one or more of further protect them, further hold them in place,

The needle card shield 280 may further comprise single-sided tape 288 or another backstop material 288. For example, the single-sided tape 288 may comprise foam tape 288. For example, the single-sided tape 288 may be approximately five inches in length. The single-sided tape 288 may be placed laterally across the needle card shield 280. The single-sided tape 288 may do one or more of further protect the needle card shield 280, further hold it in place, and further ensure its stability.

The single-sided tape 288 may be put in place above the ends or hubs of the needles 194 to act as a backstop and to help keep them from prematurely coming out of the one or more of flutes 277 and channels 277. The precise position of the single-sided tape 288 can be adjusted depending on the type of needles 194 comprised in the needle card 275. When the needle card is bent, the backstop may be bent away from the hubs so that the needles may be more easily removed.

The needle holder 193 may further comprise double-sided tape 290. For example, the double-sided tape 290 may be approximately two inches in length. The double-sided tape 290 may be placed on the needle card shield 280 so as to provide further security by the other side of the double-sided tape 290 attaching to the needle card 275. The double-sided tape 290 may do one or more of further hold it in place, and further ensure its stability.

The needle holder 193 may further comprise a needle card label 292. The needle card label 292 may comprise one or more of dosage information, an order number, a product number, manufacturer contact information, date of manufacture, location of manufacture, relevant intellectual property information including relevant information on one or more of pending patent applications and issued patents, and information on one or more of the sterility and sterilant exposure of the product.

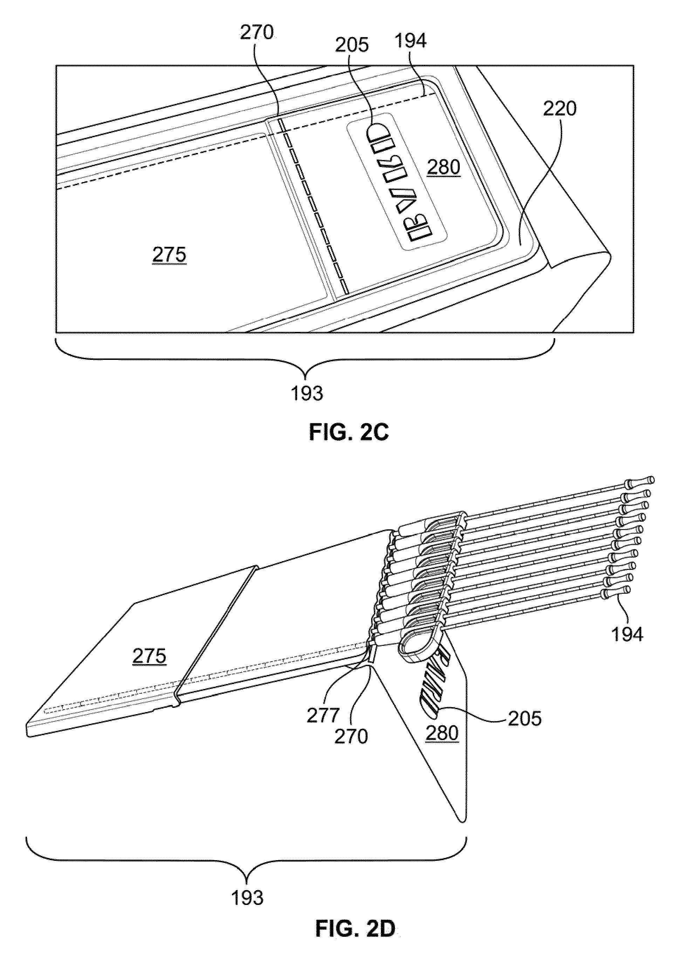

Figure 2C is a drawing showing a needle holder 193 comprising the needle card 275 and the needle card shield 280, the needle card shield 280 comprising the score 270 at a location where the needle card shield 280 may be bent in order to use it as a stand for the needle holder 193.

Figure 2C depicts a top view of the needle holder 193 after the removal (if present) of the bottom needle tray 210. The needle holder 193 comprises one or more brachytherapy needles 194. The top needle tray 220 is visible on the sides of the needle holder 193. The manufacturer name 205 that, as depicted in Figure 2C, has been stenciled on the needle holder 193 removes material from the needle holder 193. Accordingly, the manufacturer name 205 reduces the weight of the needle holder 193 by a few ounces. The needle holder 193 comprises the score 270. As depicted in Figure 2C, the score 270 is located about one-third of the way from the top of the needle holder 193 so that the needle holder 193 may stand up on its own.

Figure 2D is a drawing showing a needle holder 193 that comprises one or more brachytherapy needles 194 after the needle card shield 280 has been bent along the score 270 and used as a stand for the needle holder 193. Commonly, and as depicted in Figure 2D, though not necessarily, the needle holder comprises one or more of flutes 277 and channels 277. Commonly, though not necessarily, the needles 194 may be held loosely in the one or more of flutes 277 and channels 277 to facilitate ease of removal.

Visible on the needle card shield 280 is the stenciled manufacturer name 205. In Figure 2D, it is clear that the manufacturer name 205 that, as depicted in Figure 2C, has been stenciled on the needle holder 193, has in fact been stenciled on the needle card shield 280 and removes material from the needle card shield 280. Accordingly, the manufacturer name 205 reduces the weight of the needle card shield 280 by a few ounces and thus reduces the weight of the needle holder 193 by a few ounces. As depicted in Figure 2D, the score 270 is located about one-third of the way from the top of the needle holder 193 so that the needle holder 193 may stand up on its own.

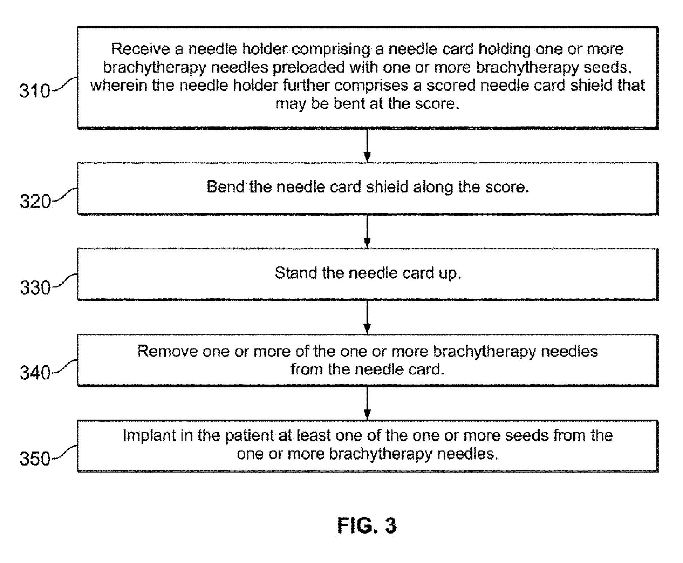

Figure 3 is a flowchart of a method 300 for treating a brachytherapy patient. The order of the steps in the method 300 is not constrained to that shown in Figure 3 nor is it constrained to that described in the following discussion. Several of the steps could occur in a different order without affecting the final result.

In block 310, a needle holder is received comprising a needle card holding one or more brachytherapy needles preloaded with one or more brachytherapy seeds, wherein the needle holder comprises a scored needle card shield that may be bent at the score. Block 310 then transfers control to block 320.

In block 320, the needle card shield is bent along the score so that the needle card stands up. Block 320 then transfers control to block 330.

In block 330, the needle card is stood up.

Block 330 then transfers control to block 340.

In block 340, one or more of the brachytherapy needles is removed from the needle card. Block 340 then transfers control to block 350.

In block 350, at least one of the one or more seeds from the one or more brachytherapy needles is implanted in the patient. Block 350 then terminates the process.

According to embodiments of the invention, a brachytherapy kit may comprise needle cards comprising one or more brachytherapy needles. According to embodiments of the invention, one or more of the one or more brachytherapy needles may be pre-loaded with one or more radioactive brachytherapy seeds. For example, one or more of the one or more brachytherapy needles may be pre-loaded with approximately one to five radioactive brachytherapy seeds.

According to embodiments of the invention, a needle card may hold one or more brachytherapy needles within one or more of flutes and channels of a die-cut corrugated sheet. According to embodiments of the invention, the needle holder comprises polyethylene. According to other embodiments of the invention, the needle holder comprises one or more of polyethylene, polypropylene, and polycarbonate. Preferably, a needle card may comprise between one and approximately forty needles. According to other embodiments of the invention, a needle card assembly may comprise between one and ten needles. According to embodiments of the invention, a needle card assembly may be individually sealed. According to other embodiments of the invention, the needles may be held loosely in the one or more of flutes and channels to facilitate ease of removal. A foam strip or other backstop material may be put in place above the needle hubs to act as a backstop and to help keep them from prematurely coming out of the one or more of flutes and channels. The precise position of the foam strip can be adjusted depending on the type of needles comprised in the needle card. When the needle card is bent, the backstop may be bent away from the hubs so that the needles may be more easily removed.

According to further embodiments of the invention, the corrugated sheet may be held on a formed powder-coated steel needle card that may provide one or more of rigidity and shielding from radiation for one or more of a physician and other medical personnel. The needles may also be secured during transport using a shrink wrap to hold securely in place one or more of the needles, the corrugated sheet, and the needle card assembly. According to other embodiments of the invention, to promote sterility and stability, the needle cards may be placed in individual thermoformed trays. According to further embodiments of the invention, to promote sterility, the needle cards may be sealed with a lid. For example, the needle cards may be sealed with a lid comprising a nonwoven product comprising spunbond olefin fiber. For example, the needle cards may be sealed with a lid comprising Tyvek, manufactured by E. I. du Pont de Nemours and Company of Wilmington, Delaware (www. dupont. com).

According to further embodiments of the invention, a number of trays may be placed within a needle box. According to other embodiments of the invention, the needle box may comprise steel. According to still further embodiments of the invention, the needle box may comprise powder-coated steel. According to yet other embodiments of the invention, the needle box may comprise a foam liner that may be configured to promote stability. According to yet further embodiments of the invention, the needle box may comprise a foam liner that may be die-cut. For example, between one and four trays may be placed within a powder-coated steel needle box.

According to other embodiments of the invention, the powder-coated steel needle box may be placed within a paperboard carton for shipping purposes. According to yet other embodiments of the invention, the carton and an approximately form-fitting insert may be placed in an outer corrugated shipping box. For example, the form-fitting insert may be a foam insert.

According to still further embodiments of the invention, the needle card may be labeled with an order number. According to yet other embodiments of the invention, a sealed needle card assembly may comprise a label. According to still other embodiments of the invention, the label may comprise one or more of dosage information, an order number, a product number, manufacturer contact information, date of manufacture, location of manufacture, relevant intellectual property information including relevant information on one or more of pending patent applications and issued patents, and information on one or more of the sterility and sterilant exposure of the product.

According to yet other embodiments of the invention, the steel box holding the needle trays may comprise a label comprising information on the radioactivity of one or more of the radioactive seeds. According to still other embodiments of the invention, the carton holding the steel box may comprise a label comprising information on the radioactivity of one or more of the radioactive seeds.

While the above representative embodiments have been described with certain components in exemplary configurations, it will be understood by one of ordinary skill in the art that other representative embodiments can be implemented using different configurations and/or different components. For example, it will be understood by one of ordinary skill in the art that the order of certain fabrication steps and certain components can be altered without substantially impairing the functioning of the invention.

For example, it will be understood by those skilled in the art that different packaging materials may be used for the needle tray. For example, the apparatus may not include a bottom needle tray 210. For example, the apparatus may not include a top needle tray 220. For example, embodiments of the invention may comprise zero shipping inserts and be configured to fit directly and snugly into the shipping box. For example, the second shipping insert 120 may comprise cavities as well as the first shipping insert 115. For example, one or more of the shipping box, the first shipping insert, and the second shipping insert may have a shape other than a right parallelepiped. For example, the cross sections of the shipping box, the first shipping insert, and the second shipping insert may be approximately circular. For example, the cross sections of the shipping box, the first shipping insert, and the second shipping insert may be approximately elliptical. For example, the cavities 117A-117H could all have different dimensions.

For example, another material may be used other than a nonwoven product comprising spunbond olefin fiber such as Tyvek. As another example, the score on the needle card allowing for the tray to be bent and to be stood up may be located near the top of the tray, i.e., less than approximately one third of the distance from the top. As another example, the score on the needle card allowing for the tray to be bent and to be stood up may be located toward the middle of the tray, i.e., more than approximately one third of the distance from the top. It is intended, therefore, that the subject matter in the above description shall be interpreted as illustrative and shall not be interpreted in a limiting sense.

The representative embodiments and disclosed subject matter, which have been described in detail herein, have been presented by way of example and illustration and not by way of limitation. It will be understood by those skilled in the art that various changes may be made in the form and details of the described embodiments resulting in equivalent embodiments that remain within the scope of the appended claims.

## Claims

1. A brachytherapy needle holder comprising a needle card holding one or more brachytherapy needles preloaded with one or more brachytherapy seeds, wherein the needle holder comprises a scored needle card shield that may be bent at the score so that the needle card may stand up on its own, providing one-handed access.

2. The brachytherapy needle holder of claim 1, wherein the needle card comprises one or more corrugations configured to hold the one or more brachytherapy needles securely in place.

3. The brachytherapy needle holder of claim 2, wherein the one or more brachytherapy needles are held within one or more of flutes and channels in the needle holder.

4. The brachytherapy needle holder of claim 3, wherein the one or more brachytherapy needles are held loosely within the one or more of flutes and channels to facilitate ease of removal.

5. The brachytherapy needle holder of claim 1, wherein the needle card shield comprises a stenciled manufacturer name.

6. The brachytherapy needle holder of claim 5, wherein the stenciled manufacturer name removes material from the needle holder, thereby reducing the weight of the needle holder.

7. The brachytherapy needle holder of claim 2, wherein the needle card is die-cut.

8. The brachytherapy needle holder of claim 2, wherein the needle card comprises one or more of polyethylene, polypropylene, and polycarbonate.

9. The brachytherapy needle holder of any preceding claim, wherein the needle card is configured to facilitate removal of one or more brachytherapy needles using one hand.

10. The brachytherapy needle holder of any preceding claim, wherein the needle card shield provides one or more of rigidity and shielding from radiation.

11. The brachytherapy needle holder of any preceding claim, wherein the needle card shield further comprises tape configured to retain the brachytherapy needles in place.

12. The brachytherapy needle holder of claim 11, wherein the tape comprises foam tape.

13. A method for removing a needle from a needle card, comprising the steps of:
receiving a needle holder comprising a needle card holding one or more brachytherapy needles preloaded with one or more brachytherapy seeds,
wherein the needle holder comprises a scored needle card shield that may be bent at the score;
bending the needle card shield along the score;
standing the needle card up; and
removing one or more of the brachytherapy needles from the needle card

14. The method of claim 13, wherein one or more of the following applies:
a) the step of standing up comprises standing the needle card up so that it stands up separately from the needle card shield, providing one-handed access,
b) the step of removing comprises removing at least one of the one or more of the brachytherapy needles with one hand, and
c) the method further comprises shielding oneself from radiation using the needle card shield.

15. A brachytherapy needle holder comprising a needle card holding one or more brachytherapy needles preloaded with one or more brachytherapy seeds, wherein the needle holder further comprises a needle card shield that provides one or more of rigidity and shielding from radiation for one or more of a physician and other medical personnel.
